# EUROPEAN PATENT APPLICATION

(11) **EP 4 732 777 A1**
(43) Date of publication of application: **29.04.2026**
(21) Application number: 24208685.8
(22) Date of filing: 24.10.2024
(51) Int. Cl.: A61B 6/06, A61B 6/08, A61B 6/00

(54) **CONTROLLING AN X-RAY RADIATION FIELD ON AUGMENTED LIVE VIDEO USING 3D INFORMATION**

(71) Applicant: Koninklijke Philips N.V., 5656 AG Eindhoven (NL)
(72) Inventor: SCHLUETER, Mathias, Eindhoven (NL)
(74) Representative: Philips Intellectual Property & Standards

(57) **Abstract**

Disclosed is a method (100) for controlling an x-ray system (102), wherein the x-ray system (102) is configured to acquire x-ray images (142) of a target radiation field (134) based on imaging instructions (132) provided by an operator (500) on a display unit (130), wherein the size of the target radiation field (134) may exceed a size of a detector (116). The display unit (130) is configured to display augmented image data (120), wherein the augmented image data (128) comprises 3D projection information (124) about the position of the subject (106) as well as overlays of virtual collimation fields (136). The virtual collimation fields (136) comprise sets of virtual positions (138) of the x-ray components (110). The x-ray system (102) may then be controlled to move the x-ray components (110) to the virtual positions (138), acquiring an x-ray image (142) at each virtual position (138).

## Description

### FIELD OF THE INVENTION

The invention relates to the field of radiation imaging, and in particular to the field of x-ray projection imaging techniques.

### BACKGROUND

The acquisition of x-ray images requires an accurate alignment of various components of an x-ray system, in particular an alignment of positions and orientations of an x-ray tube, a collimator and a detector relative to a subject positioned on a subject support. To obtain an accurate alignment, an operator may manually align these components with regards to target radiation field, wherein the target radiation field indicates an area of the subject to be imaged, or the components may be automatically moved to a target position.

In particular in stitching examinations, for example examinations of large areas of the body such as the spine or legs of the subject, multiple partial x-ray images may need to be acquired to fully cover the target radiation field, as the target radiation field may exceed a size of the x-ray detector and/or a size of a collimation field defined by the collimator. In such examinations, an operator may need to manually readjust the components of the x-ray system after the acquisition of each partial x-ray image.

The patent application US 2017 100 089 A1 describes a method and apparatus for x-ray tube scanner automation using a 3D camera. An RGBD image of a patient on a patient table is received from a 3D camera mounted on an x-ray tube, wherein the RGBD image may include a detection of ring markers on the patient table. A transformation between a coordinate system of the 3D camera and a coordinate system of the patient table is calculated. In particular, the detected ring markers may be used for the calibration of the RGBD image to the coordinate system of the patient table. A patient model is estimated from the RGBD image of the patient. The x-ray tube is automatically controlled to acquire an x-ray image of a region of interest of the patient based on the patient model.

### SUMMARY OF THE INVENTION

The invention provides for a method for controlling an x-ray system, an x-ray system and a computer program product in the independent claims. Embodiments are given in the dependent claims. The objectives underlying the invention are solved by the features of the independent claims.

In one aspect, the invention provides for a method for controlling an x-ray system, wherein the x-ray system comprises a subject support configured for receiving a subject, a camera system and x-ray components comprising an x-ray tube, a collimator mounted on the x-ray tube and a detector, and wherein the x-ray system defines a collimation field.

The x-ray components are configured to move in a planar translational motion along a plane defined by the subject support, wherein the x-ray tube and the collimator are further configured to move in a rotational motion around an axis parallel to the plane such that the collimation field is also translated along the plane. The camera system is configured to provide image data of at least part of the subject support in a camera image zone larger than the collimation field.

The method comprises receiving the image data, wherein the image data comprises visual information and depth information.

The method further comprises calculating 3D projection information specifying a position of the subject on the subject support relative to a position of the x-ray components using the depth information, the position of the x-ray components being described by position information.

The method further comprises providing augmented image data on a display unit, the augmented image data comprising images rendered from the visual information and the 3D projection information.

The method further comprises receiving imaging instructions from the display unit, the imaging instructions defining a target radiation field.

The method further comprises determining one or multiple virtual collimation fields covering together the target radiation field, the target radiation field being equal in size or larger than an individual one of the virtual collimation fields, wherein each virtual collimation field is associated with a set of virtual positions of the x-ray components based on the 3D projection information and the position information.

The method further comprises moving the x-ray components in the planar translational motion and moving the x-ray tube and the collimator in the rotational motion to the sets of virtual positions.

In another aspect, the invention provides for an x-ray system, wherein the x-ray system comprises a subject support configured for receiving a subject, a camera system and x-ray components comprising an x-ray tube, a collimator mounted on the x-ray tube and a detector, the x-ray system defining a collimation field.

The x-ray components of the x-ray system are configured to move in a planar translational motion along a plane defined by the subject support, wherein the x-ray tube and the collimator are further configured to move in a rotational motion around an axis parallel to the plane such that the collimation field is also translated along the plane. The camera system is configured to provide image data of at least part of the subject support in a camera image zone larger than the collimation field.

The x-ray system further comprises a computer system, wherein the computer system comprises a memory unit storing machine executable instructions, wherein execution of the machine executable instructions causes the computer system to receive the image data, the image data comprising visual information and depth information, and position information of the x-ray components.

Execution of the machine executable instructions further causes the computer system to calculate 3D projection information specifying a position of the subject on the subject support relative to a position of the x-ray components using the depth information, the position of the x-ray components being described by position information.

Execution of the machine executable instructions further causes the computer system to provide augmented image data on a display unit, the augmented image data comprising images rendered from the visual information and the 3D projection information.

Execution of the machine executable instructions further causes the computer system to receive imaging instructions from the display unit, the imaging instructions defining a target radiation field.

Execution of the machine executable instructions further causes the computer system to determine one or multiple virtual collimation fields covering together the target radiation field, the target radiation field being equal in size or larger than an individual one of the virtual collimation fields, wherein each virtual collimation field is associated with a set of virtual positions of the x-ray components based on the 3D projection information and the position information.

Execution of the machine executable instructions further causes the computer system to move the x-ray components in the planar translational motion and move the x-ray tube and the collimator in the rotational motion to the sets of virtual positions.

In another aspect, the invention provides for a computer program product, wherein the computer program product comprises machine executable instructions configured for causing an x-ray system, to execute the inventive method.

### BRIEF DESCRIPTION OF THE DRAWINGS

In the following, examples are described in greater detail making reference to the drawings in which:
Fig. 1 depicts a schematic of an x-ray system according to the present subject matter,
Fig. 2A shows a flowchart which illustrates a method for controlling the x-ray system according to the present subject matter,
Fig. 2B shows a flowchart which illustrates a method for controlling the x-ray system according to the present subject matter,
Fig. 3 depicts a schematic of exemplary augmented image data, including multiple overlapping virtual collimation fields overlayed over a subject on a subject support,
Fig. 4A depicts a schematic of exemplary augmented image data, including a collimation field and multiple overlapping virtual collimation fields overlayed over the subject on the subject support,
Fig. 4B depicts a schematic of exemplary augmented image data, including a collimation field at a central position of a target radiation field and multiple overlapping virtual collimation fields overlayed over the subject on the subject support,
Fig. 5 depicts a schematic of a calculating of 3D projection information,
Fig. 6 shows a flowchart of the calculating of the 3D projection information,
Fig. 7 depicts a block diagram of an exemplary computer system for implementing at least part of the present method in accordance with an example of the present subject matter.

### DETAILED DESCRIPTION

Like numbered elements in these figures are either equivalent elements or perform the same function. Elements which have been discussed previously will not necessarily be discussed in later figures if the function is equivalent.

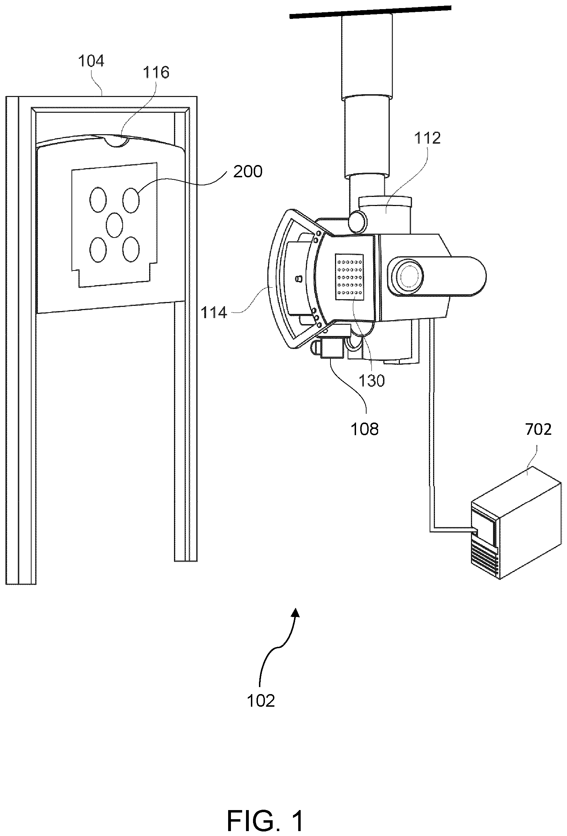

Fig. 1 depicts a schematic of an x-ray system 102 according to the present subject matter. The x-ray system 102 comprises x-ray components 110, the x-ray components comprising an x-ray tube 112, a collimator 114 and a detector 116. The x-ray system 102 further comprises a camera system 108, a subject support 104, a computer system 702 and a display unit 130.

In one example, the x-ray tube 112 comprises an x-ray source configured for generating x-rays and a window, wherein the x-rays generated by the x-ray source may exit the x-ray tube through the window. The x-ray tube may be covered by a housing of the x-ray tube. The housing may exemplarily comprise a metallic housing, such as a housing of lead-lined steel. The housing serves to prevent leakage of x-ray radiation. The x-ray tube 112 may further comprise an x-ray tube support covering the x-ray source and the housing, wherein the x-ray tube support enables a mechanical mounting of the x-ray tube 112. The mechanical mounting of the x-ray tube 112 may exemplarily comprise a mechanical mounting of the x-ray tube support on a ceiling of a room the x-ray system 102 is located in, as shown in Fig.1. In another example, the mechanical mounting of the x-ray tube 112 comprises a mechanical mounting of the x-ray tube support on a floor-mounted stand. The x-ray tube support 112 may further be configured for mechanically mounting the collimator 114 and/or the camera system 108 on the x-ray tube 112 and/or the x-ray tube support. In another example, the camera system 108 is mechanically mounted on the collimator 114, wherein the collimator 114 is mounted on the x-ray tube 112. According to this example, the camera system 108 is configured to perform rotational movements in accordance with rotational movements of the collimator 114.

The collimator 114 is mounted to the x-ray tube, for example on the x-ray tube support. The collimator 114 comprises collimation blades, wherein the collimator 114 is configured to open or close at least one of the collimation blades in response to receiving a signal from the computer system 702. The collimator 114 collimates the x-rays generated by the x-ray source of the x-ray tube 112 and directs the x-rays towards a collimation field 118. The collimation field 118 indicates an area in a plane defined by the subject support 104, wherein x-rays generated by the x-ray source of the x-ray tube 112 are configured to irradiate the collimation field 118.

The detector 116 is configured to detect incidences of x-rays generated by the x-ray source of the x-ray tube 112 at particular locations of the detector. The detector 116 is further configured to send a detection signal to the computer system 702 in response to the detecting of x-rays, wherein the detection signal exemplarily comprises intensity values for the detected x-rays for a set of locations of the detector 116. The detector 116 may exemplarily be comprised by a detector support system 116. For example, the detector support system 116 may comprise a housing of the detector 116, wherein the housing of the detector 116 may comprise a metallic and/or plastic housing. The detector support system 116 is configured to be mechanically mounted on the subject support 104. The mechanical mounting of the detector support system 116 may enable the detector 116 to perform linear translational movements along the plane defined by the subject support 104. The detector support system 116 exemplarily comprises the detector 116 and at least one automatic exposure control chamber (AEC chamber), wherein the at least one AEC chamber is configured to measure an amount of x-rays received from the x-ray tube 112 and send a satiation signal to the computer system 702 upon detecting an amount of x-rays exceeding a satiation threshold. In response to receiving the satiation signal, the computer system 702 is configured to stop the generating of x-rays by the x-ray source of the x-ray tube 112. The at least one AEC chamber is configured to measure an amount of x-rays received in a partial area of the detector 116, wherein the partial area is indicated by an amplimat field 200 of the detector 116. For example, the detector may comprise five amplimat fields 200 as shown in Fig. 1, including a central amplimat field 200 surrounded by four further amplimat fields 200 in four corners of the detector 116.

The x-ray system 102 further comprises the camera system 108. The camera system 108 may exemplarily comprise a 3D camera or a set of multiple 2D cameras. The camera system comprises a sensor plate configured for receiving image data 120 of at least part of the subject support 104 in a camera image zone 122 larger than the collimation field 118, and wherein the camera system is further configured to provide the image data 120. The image data 120 is provided on the display unit 130, wherein the display unit 130 may exemplarily be mounted on the x-ray tube 112 as shown in Fig. 1. Alternatively, the camera system 108 may be mounted on the collimator 114. In an alternative example, the display unit may be a display unit at a location separate from the x-ray tube 112. The display unit 130 and the camera system 108 are communicatively coupled with the computer system 702. In Fig. 1, the camera system 108 exemplarily comprises a 3D camera, wherein the 3D camera is mounted on the x-ray tube 112. In an alternative example, the camera system 108 may be mounted at another fixed position, for example, on a wall or ceiling of a room the x-ray system 102 is located in.

The x-ray components 110 are communicatively coupled with the computer system 702 of the x-ray system 102. The communicative coupling enables the x-ray components 110 to send signals to the computer system 702 and receive signals from the computer system 702. The computer system is configured to send signals to the x-ray components resulting in movements of the x-ray components.

In particular, the x-ray tube 112 is configured to move in a planar translational motion along a plane defined by the subject support 104 such that the collimation field 118 is also translated along the plane. Said plane defined by the subject support 104 exemplarily comprises a plane parallel to the subject support 104 and intersecting with the subject support 104 or a plane parallel to the subject support 104 located at a fixed distance to the subject support 104. In one example, the x-ray tube 112 may be further configured to move in a linear translational motion along an axis orthogonal to the plane defined by the subject support 104, wherein said moving in a linear translational motion results in an increase or decrease of a distance between the x-ray tube 112 and the subject support 104. The x-ray tube 112 is further be configured to move in a rotational motion around an axis parallel to the plane defined by the subject support 104 such that the collimation field 118 is also translated along the plane.

The collimator 114 is mounted on the x-ray tube 112. The collimator 114 configured to move in the planar translational motion along a plane defined by the subject support 104 along with the x-ray tube 112 such that the collimation field 118 is also translated along the plane. The collimator 114 is further configured to move in the rotational motion around an axis parallel to the plane defined by the subject support 104 along with the x-ray tube 112 such that the collimation field 118 is also translated along the plane. In one example, the rotational motion of the collimator 114 around the axis parallel to the plane defined by the subject support 104 may be performed due to the mechanical mounting of the collimator 114 to the x-ray tube 112 in response to a rotational motion of the x-ray tube 112 around the axis parallel to the plane defined by the subject support 104. In one example, the collimator 114 is further configured to move in a rotational motion around an axis orthogonal to the plane defined by the subject support 104 independent of the x-ray tube 112, such that the collimation field 118 is also rotated inside the plane.

The detector 116 is configured to move in a planar translational motion along the plane defined by the subject support 104. For example, the detector 116 may be mechanically mounted onto the subject support 104 as shown in Fig. 1, wherein the subject support 104 is configured for receiving the subject 106 in a location between the detector 116 and the x-ray tube 112 such that x-rays generated by the x-ray source of the x-ray tube pass through the subject 106 towards the detector 116. The subject support 104 may comprise a wall stand as shown in Fig. 1, wherein the wall stand is configured for receiving the subject 106 in a standing position. The subject 106 is not shown in Fig. 1 for visual clarity. In an alternative example, the subject support 104 may comprise a table, wherein the table is configured for receiving the subject 106 in a supine or prone position.

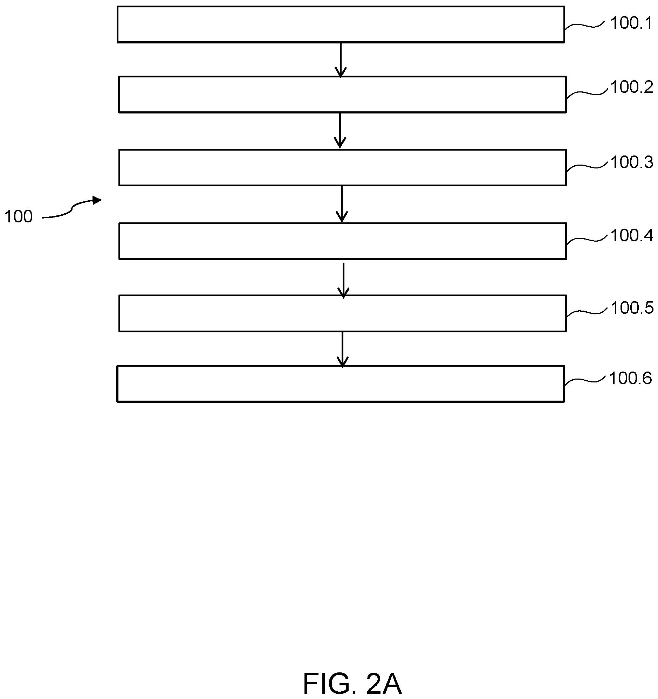

Fig. 2A shows a flowchart which illustrates a method 100 for controlling the x-ray system according to the present subject matter.

Before performing the method 100 shown in Fig. 2A, the x-ray system 102 may be calibrated in a setup step. The setup step exemplarily comprises an adjustment of a distance between the x-ray tube 112 and the subject support 104 by moving the x-ray tube 112 in a linear translational motion along an axis orthogonal to the subject support 104. The setup step may further comprise moving the x-ray tube 112 and/or the collimator in a rotational motion, such that the collimation field 118 is also rotated inside the plane defined by the subject support 104. Said rotational motion of the collimator 114 may be a rotational motion of the collimator 114 along with the rotational motion of the x-ray tube 112 resulting from the mounting of the collimator 114 on the x-ray tube, or a rotational motion of the collimator 114 independent of the rotational motion of the x-ray tube 112.

In step 100.1, the image data 120 is received by the computer system 702 of the x-ray system 102 by the camera system 108, wherein the image data comprises visual information 120.1 and depth information 120.2. The visual information may exemplarily comprise RGB values for pixels in a two-dimensional image, wherein the depth information 120.2 comprises a depth value for every pixel in the two-dimensional image. The two-dimensional image may for example be a two-dimensional image of a set of two-dimensional images in a video stream.

In step 100.2, 3D projection information 124 specifying a position of the subject 106 on the subject support 104 relative to a position of the x-ray components 110 is calculated using the depth information 120.2, wherein the position of the x-ray components 110 is described by position information 126. The 3D projection information 124 exemplarily comprises a set of three-dimensional points, wherein coordinates of the three-dimensional points correspond to the position of the subject 106 on the subject support 104. The position of the subject 106 as used herein refers to points on a surface of the subject 106 as provided by the image data 120.

In one example, the position information 126 comprises location information 126.1 providing coordinates in 3D space and orientation information 126.2 providing angles of orientation of the x-ray components 110. The position information 126 may exemplarily be provided by position sensors of the x-ray components, wherein the position sensors are communicatively coupled with the computer system 702 of the x-ray system, and the position information 126 is stored in the memory unit 705 of the computer system 702. In one example, the position sensors comprise accelerometers and/or magnetometers configured to provide the orientation information 126.2 of the x-ray components. According to this example, the location information 126.1 may be determined from a previous location information by using the accelerometers for displacement tracking, wherein acceleration data provided by the accelerometer is integrated to obtain velocity data and the velocity data is subsequently integrated to obtain the position information 126.1. According to this example, a reference location information 126.1 may be provided by the computer system 702, wherein the reference location information 126.1 is determined in a location calibration step. The location information 126.1 may be updated after any movements of the x-ray components occurring after the location calibration step, wherein the location information 126.1 is updated in the memory unit 705 of the computer system 702. In another example, the position sensors comprise GPS transmitters configured to provide the location information 126.1 as GPS data. The location information 126.1 and/or the orientation information 126.2 may exemplarily be provided to the computer system 702 by the position sensors using a wired connection between the computer system 702 and the position sensors or a wireless connection between the computer system 702 and the position sensors, for example a Wi-Fi connection or a Bluetooth connection.

In step 100.3, augmented image data 128 is provided on the display unit 130, the augmented image data 128 comprising images rendered from the visual information 120.1 and the 3D projection information 124. In one example, the augmented image data 128 is provided on the display unit 130 as a live video stream of the camera image zone 122.

In step 100.4, imaging instructions 132 are received from the display unit 130, wherein the imaging instructions 132 define a target radiation field 134. The imaging instructions 132 may exemplarily be received by the computer system 702 of the x-ray system 102 in response to an input provided on the display unit 130 by an operator 500. The target radiation field 134 defines a desired total area of the subject 106 on the subject support 104 to be irradiated using the x-rays generated by the x-ray source of the x-ray tube 112.

In step 100.5, one or multiple virtual collimation fields 136 covering together the target radiation field 134 are determined, the target radiation field 134 being equal in size or larger than an individual one of the virtual collimation fields 136, wherein each virtual collimation field 136 is associated with a set of virtual positions 138 of the x-ray components 110 based on the 3D projection information 124 and the position information 126.

The virtual collimation fields 136 indicate projected positions of the x-ray components 110, wherein the projected positions are projected from the x-ray tube 112. For example, if the x-ray tube 112 is displaced relative to the camera system 108 and/or the x-ray tube 112 is rotated at an angle relative to an axis orthogonal to the detector 116, positions of the x-ray components 110 as positioned relative to a coordinate system of the camera system 108 and displayed in the image data 120 may differ from positions of the x-ray components as positioned relative to a coordinate system of the x-ray tube 112. The use of the 3D projection information 124 calculated using the position information 126 enables for the determining of the virtual collimation fields 136, wherein the virtual collimation fields 136 accounts for said differences between the coordinate system of the camera system 108 and the coordinate system of the x-ray tube 112. Advantageously, the virtual collimation fields 136 and the associated virtual positions 138 may be defined in particular for virtual positions 138 of the x-ray tube 112 which differ from an actual position of the x-ray tube 112.

The virtual positions 138 of the x-ray components 110 may also be referred to as prospective positions of the x-ray components 110. The set of virtual positions 138 of the x-ray components 110 comprises a set of virtual positions of the x-ray tube 112, a set of virtual positions of the collimator 114 and a set of virtual positions of the detector 116. Each set of virtual positions 138 of one of the x-ray components 110 indicates a virtual location as well as a virtual orientation in space of the x-ray component 110. For example, a virtual position 138 of the collimator 114 may comprise coordinates indicating a location of the collimator 114 in three-dimensional space and an angle of orientation of the collimator 114. The coordinates in three-dimensional space may be defined relative to an origin of a fixed coordinate system, for example a coordinate system of the x-ray tube 112, a coordinate system of the collimator 114 or a coordinate system of the camera system 108. The angle of orientation may be defined relative to a fixed reference orientation, wherein the fixed reference orientation may be provided by the computer system 702, and wherein the fixed reference orientation may for example correspond to an orientation of a gravitational field of the earth. The sets of virtual positions 138 are stored in a memory unit 705 of the computer system 702. The virtual collimation fields 136 may exemplarily be indicated by overlays in the augmented image data 128 on the display unit 130.

In step 100.6, the x-ray components 110 move in the planar translational motion and the x-ray tube 112 and the collimator 114 move in the rotational motion to the sets of virtual positions 138. After the moving 100.6 of the x-ray components to a particular set of virtual positions 138, the actual positions of the x-ray components 110 will correspond to the virtual positions 138 of the x-ray components 110. After the moving 100.6, the collimation field 118 will correspond to the virtual collimation field 136 associated with the particular set of virtual positions 138. The moving 100.6 of the x-ray components 110 may for example result from a movement signal sent by the computer system 702 towards motors of the x-ray components 110, wherein the movement signal causes the motors of the x-ray components 110 to move in translational and/or rotational motions such that actual positions of the x-ray components 110 are aligned with a set of virtual positions 138. The motors of the x-ray components 110 may for example be electric motors, in particular stepper motors and/or servo motors.

The method 100 may enable a more precise automatic control of movements of the x-ray components 110. The moving 100.6 of the x-ray components in an automated manner may result in a more precise covering of the target radiation field 134 by aligning the collimation field 118 with the one or multiple virtual collimation fields 136. Furthermore, the operation of the x-ray 102 may be simplified, as the positions of the x-ray components 110 are prospectively defined by the virtual positions 138 before moving 100.6 the x-ray components 110. Therefore, there is no need for the operator 500 of the x-ray system 102 to intervene or manually adjust the x-ray components 110 to align the collimation field 118 with the one or multiple virtual collimation fields 136. In particular, the prospective defining of the positions of the x-ray components 110 by the virtual positions 138 may be beneficial when a position of the x-ray tube 112 is located at an inconvenient height during an x-ray scan, for example when the target radiation field 134 encompasses a foot and/or leg of the subject 106.

The method 100 may be further advantageous for performing x-ray scans on an asymmetric target radiation field 134, and in particular a target radiation field 134 larger than the collimation field 118, as the target radiation field 134 may comprise multiple virtual collimation fields 136. The target radiation field 134 may be defined by the operator 500 on the display unit 130 as an overlay on the augmented image data 128, wherein the imaging instructions 132 provided by the operator 500 are converted into geometrical information of positions and orientations of the x-ray components 110 by the computer system 702 using the 3D projection information, and wherein the geometrical information is converted into movement commands for the x-ray components 110 by the computer system 702.

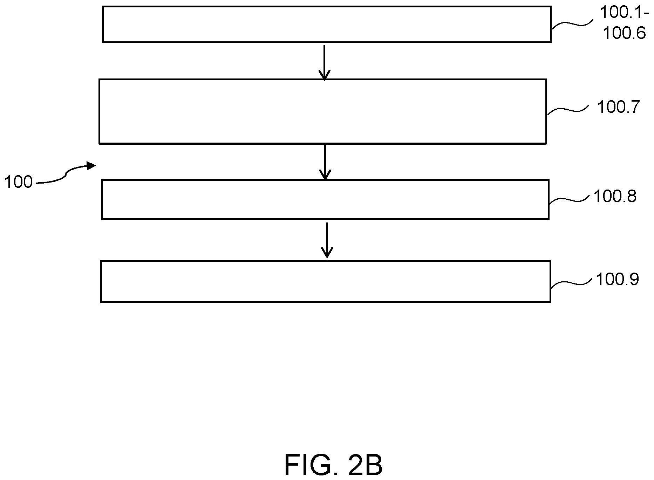

Fig. 2B shows a flowchart which illustrates the method for controlling the x-ray system according to the present subject matter.

In one example, the method further comprises: for the sets of virtual positions 138, performing an x-ray scan of the subject 106 using the virtual collimation field 136 actually defined by the set of virtual positions 138 in step 100.7, the x-ray scan resulting in a respective x-ray dataset 140 per set of virtual positions 138, constructing 100.8 at least one x-ray image 142 of the subject 106 based on the x-ray datasets 140 and the sets of virtual positions 138 in step 100.8, and providing 100.9 the at least one x-ray image 142 in step 100.9.

The performing 100.7 of the x-ray scan exemplarily comprises sending a start signal from the computer system 702 of the x-ray system 102 to the x-ray tube 112, wherein the start signal causes the generating of x-rays by the x-ray source of the x-ray tube 112. The x-rays then irradiate the collimation field 118 corresponding to a particular virtual collimation field 136, such that the x-rays pass through an area of the subject 106 inside the collimation field 118 and reach the detector 116, causing the detector 116 to send the detection signal to the computer system 702. The computer system is configured to generate the x-ray data 140 for the particular virtual collimation field 136 from the intensity values for the set of locations of the detector 116 from the detection signal and the sets of virtual positions 138 of the x-ray components 110 associated with the particular virtual collimation field 136.

In one example, the constructing 100.8 of the x-ray image 142 comprises a stitching of multiple x-ray images 142 resulting from the performing of multiple x-ray scans on multiple collimation fields 118 covering the target radiation field 134 into a stitched x-ray image 142', and providing the x-ray image 142 as the stitched x-ray image 142'. The constructing 100.8 of the at least one x-ray image 142 of the subject 106 may exemplarily comprise converting the intensity values for the set of locations of the detector 116 from the detection signal into an image, for example by converting intensity values into greyscale values of the at least one x-ray image 142.

The performing of x-ray scans for providing x-ray images 142 of a target radiation field 134 larger than the collimation field 118 according to the method 100 may be beneficial for stitching examinations, wherein the target radiation field 134 may exemplarily extend over a spine or a leg of the subject 106. The method 100 simultaneously enables a decreasing of a time between acquiring individual x-ray images 142, as the target radiation field 134 only needs to be defined once by the imaging instructions 132 received in step 100.4. Following the defining of the target radiation field 134, any following x-ray scans may be performed 100.7 in a single automated imaging sequence without requiring readjustment of the x-ray components 110 by the operator 500 after performing an x-ray scan on a collimation field 118 corresponding to any particular virtual collimation field 136. The virtual collimation fields 136 further enable an assessment of multiple prospective collimation fields 118 corresponding to the virtual collimation fields 136 at the same time. This may advantageously improve a quality of the provided x-ray images 142. The quality of the provided x-ray images 142 may be further improved as movement artifacts arising from movements of the subject 106 during the performing 100.7 of the x-ray scans may be minimized. This may be particularly beneficial for performing x-ray scans on subjects prone to movement during an examination, such as children. As the virtual position 138 indicate the positions of the x-ray components during the performing 100.7 of the x-ray scan, the stitching of the multiple x-ray images 142 may be simplified as an area in one of the multiple x-ray images 142 may be more easily matched to an area in another one of the multiple x-ray images 142 using the virtual positions 138.

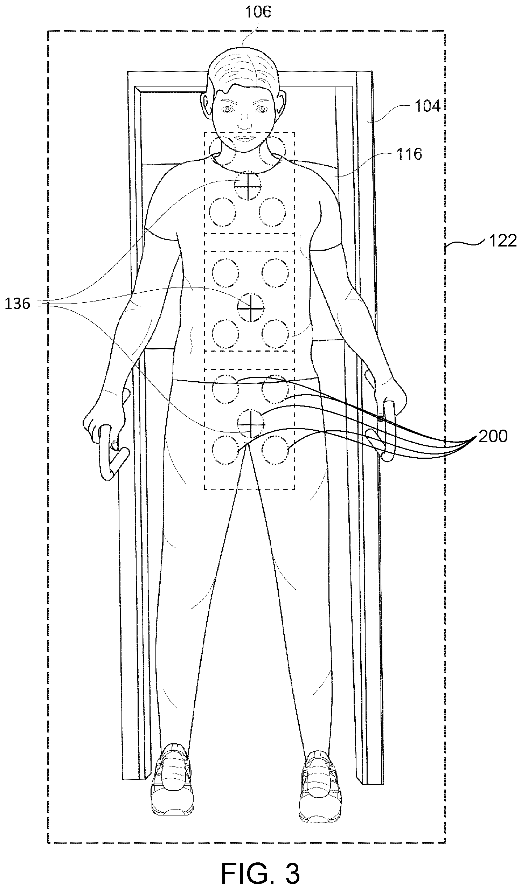

Fig. 3 depicts a schematic of exemplary augmented image data 128 displaying the camera image zone 122, including multiple overlapping virtual collimation fields 136 overlayed over the subject 106 on the subject support 104.

In one example, the virtual collimation fields 136 cover the target radiation field 134 in a contiguous and/or overlapping manner. According to this example, a size of the target radiation field 134 may further exceed a size of the detector 116.

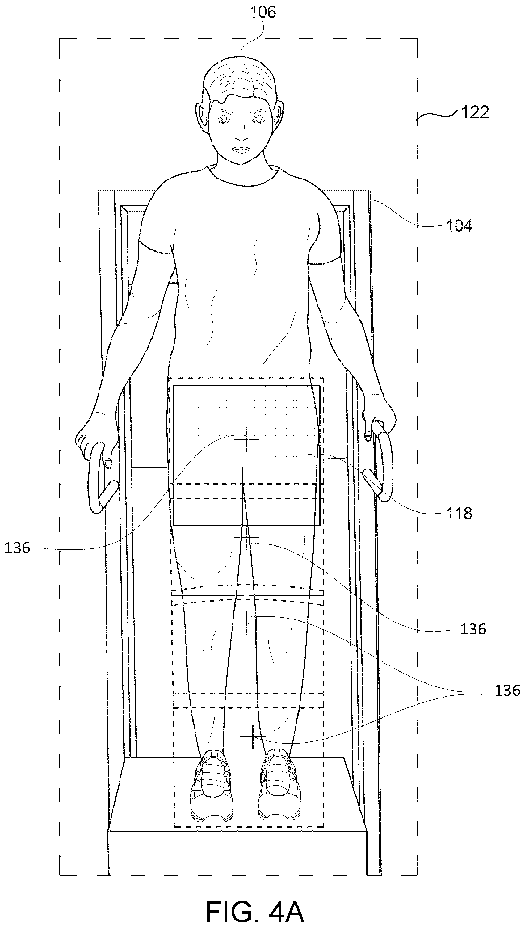

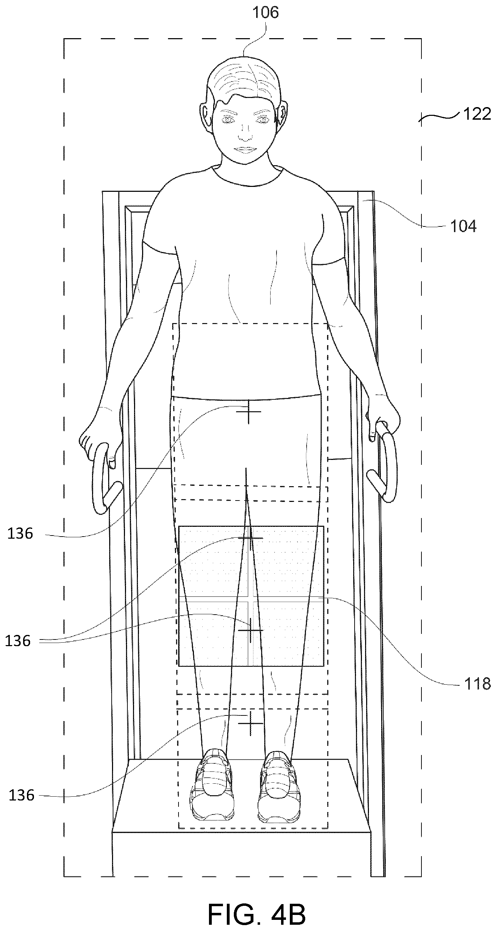

In Fig. 3, as well as in the following Figs 4A and 4B, the target radiation field 134 is defined by an outer contour of multiple virtual collimation fields 136, wherein the virtual collimation fields 136 are partially overlapping. In an alternative example, the target radiation field 134 may be defined by an outer contour of a single virtual collimation field 136. In a further alternative example, the target radiation field 134 may be defined by an outer contour of multiple virtual collimation fields 136, wherein the virtual collimation fields are contiguous.

The virtual collimation fields 136 may for example be indicated as rectangular shapes with dashed outlines on the augmented image data 128 displayed on the display unit 130, as shown in Fig. 3. A central position of the virtual collimation fields 136 may optionally be indicated by a cross shape as shown in Fig. 3.

Overlays of the virtual collimation fields 136 may be advantageous compared to, for example, optical light fields projected onto the subject 106 to indicate the collimation field 118. The overlays may display all virtual collimation fields 136 simultaneously. The overlays may further be displayed on any display unit 130, for example a remote display unit 130 in a room the x-ray system 102 is located in. Furthermore, overlays of the virtual collimation fields 136 are more accurate with respect to the irradiated collimation field 118 than said optical light fields, as said optical light fields may contain mechanical inaccuracies of mounted components such as mirrors used to generate said optical light fields.

In one example, the imaging instructions 132 further comprise instructions for activating or deactivating amplimat fields 200 of the detector 116 for the virtual collimation fields 136, wherein the x-ray scan is performed 100.7 only using activated amplimat fields 200.

The amplimat fields 200 allow for automatic exposure control (AEC) of the x-ray system 102. For example, if an upper half of a virtual collimation field 136 is overlayed over the subject 106, while a lower half of the virtual collimation field is not overlayed over the subject 106, x-rays passing through the lower half would not be attenuated by passing through the subject 106 before hitting the detector 116. This may for example result in a satiation signal being sent to the computer system 702, resulting in a premature stop of an x-ray scan. Advantageously, the amplimat fields 200 may be display as virtual amplimat fields 200 associated with virtual positions 138 of the detector 116 for the virtual collimation fields 136. A receiving of an input on the display unit 130 may result in an activating or deactivating of the virtual amplimat fields 200. In Fig. 1, the virtual amplimat fields 200 are exemplarily indicated as circular shapes with dashed outlines for the virtual collimation fields 136. While the virtual amplimat fields 200 are not shown in the following Fig. 4A and Fig. 4B for visual clarity, the display of the virtual amplimat fields 200 may be combined with the examples shown in Fig. 4A and Fig. 4B.

The amplimat fields 200 correspond to partial areas of the detector 116, wherein only the partial areas of the detector 116 corresponding to activated amplimat fields 200 are used during the performing 100.7 of x-ray scans. The virtual amplimat fields 200 shown in the augmented image data 128 enable a prospective activating or deactivating of the amplimat fields 200 for multiple virtual collimations fields 136 before performing 100.7 the x-ray scans, enabling automatic exposure control for prospective virtual positions 138 of the x-ray components 110.

Fig. 4A depicts a schematic of exemplary augmented image data 128, including a collimation field 118 and multiple overlapping virtual collimation fields 136 overlayed over the subject 106 on the subject support 104. For example, the collimation field 118 shown in Fig. 4A may result from positions of the x-ray components 110 before the receiving 100.4 of the imaging instructions 132 defining the target radiation field 134.

In one example, the target radiation field 134 may be indicated directly on the augmented image data 128 displayed on the display unit 130, for example by input received from the operator 500. Advantageously, the target radiation field may be adjusted on the augmented image data 128 displayed on the display unit 130 in real time. A number of virtual collimation fields 136 may automatically appear or disappear as overlays on the augmented image 128 such that the target radiation field 134 is covered by the virtual collimation fields.

Fig. 4B depicts a schematic of exemplary augmented image data 128, including a collimation field 118 at a central position of a target radiation field 134 and multiple overlapping virtual collimation fields 136 overlayed over the subject 106 on the subject support 104. The virtual collimation fields 136 may overlap depending on a size of the target radiation field relative to a size of the virtual collimation fields 136. For example, Fig. 4A shows three virtual collimation fields 136 covering the target radiation field. According to this example, if the size of the target radiation field 134 were increased in response to an input on the display unit, the virtual collimation fields 136 would change their positions to adjust to the increased size of the target radiation field 134, thereby reducing a size of overlapping areas between the virtual collimation fields 136. If the size of the target radiation field 134 were increased beyond the size of the three virtual collimation fields 136, a fourth virtual collimation field 136 would appear on the augmented image data 128 to cover the target radiation field 134 while adjusting the positions of all four virtual collimation fields 136, as shown in Fig. 4B. The virtual collimation fields 136 advantageously enable an assessment of overlapping areas between x-ray images 142 provided 100.9 by performing 100.7 x-ray scans on the virtual collimation fields 136.

In one example, the moving 100.6 of the x-ray components 110 to the virtual positions 138 comprises moving the x-ray tube 112 in a planar translational motion to a central position of the target radiation field 134, and maintaining the position of the x-ray tube 112 at the central position of the target radiation field 134. According to this example, the moving 100.6 further comprises for the sets of virtual positions 138: moving the detector 116 in a planar translational motion to the respective actual virtual position 138 of the detector 116, and rotating the x-ray tube 112 and the collimator 114 to align with the virtual collimation field 136 actually defined by the set of virtual positions 138.

According to this example, the x-ray tube 112 only performs a single movement towards the central position of the target radiation field 134 during the performing of the method 100, such that the collimation field 118 of the x-ray tube 112 after the single movement to the central position is aligned with the central position of the target radiation field 134 as displayed on the augmented image data 128. The maintaining of the position of the x-ray tube 112 at the central position of the target radiation field 134 advantageously minimizes a tilt angle of the x-ray tube 112 during the performing 100.7 of x-ray scans on multiple collimation fields 136. This advantageously minimizes an obliqueness of x-rays originating from the x-ray tube 112 relative to the subject support 104 during the performing 100.7 of the x-ray scans. A further advantage according to this example may be a saving of operation time since the operator 500 need not move the x-ray tube 112 manually to the central position of the target radiation field 134, further enabling the x-ray tube 112 to stay at a convenient operation height.

The single movement of the x-ray tube 112 towards the central position of the target radiation field 134 may be determined by the computer system 702 by geometrical calculations, wherein the geometrical calculations use the 3D projection information 124 specifying the position of the subject 106 relative to actual positions of the x-ray tube 112 and the collimator 114.

In one example, the x-ray scan is performed 100.7 on an uppermost virtual collimation 136 field shown in Fig. 4B, that is the virtual collimation field 136 closest to the upper body of the subject 106, the x-ray tube 112 may first move to the central position of the target radiation field 134, resulting in the collimation field 118 shown in Fig. 4B. Subsequently, the collimator 114 and the detector 116 may be moved to the virtual positions 138 of the collimator 114 and the detector 116 associated with the uppermost virtual collimation field 136. This may exemplarily comprise moving the collimator in a rotational motion towards the upper body of the subject 106 and moving the detector in a translational motion towards the upper body of the subject 106.

In one example, the image data 120 comprises an RGB video data stream, in particular a live video feed, and the camera system 108 comprises a 3D camera or a set of 2D camera. In one example, the camera system 108 comprises a 3D camera mounted on the x-ray tube 112 or the collimator 114, wherein the 3D camera is configured for providing image data 120 comprising visual information 120.1 and depth information 120.2. In another example, the camera system 108 comprises a set of 2D cameras, wherein each 2D camera in the set of 2D cameras is configured to image data comprising visual information 120.1, and wherein depth information 120.2 may be determined by the computer system 702 using the visual information 120.1 and position information 126 of the set of 2D cameras. For example, the depth information 120.2 according to this example may be determined by an image processing algorithm configured to detect an object in the visual information 120.2 of at least two 2D cameras of the camera system 108 and determine a position of the object using the position information 126 of the at least two 2D cameras.

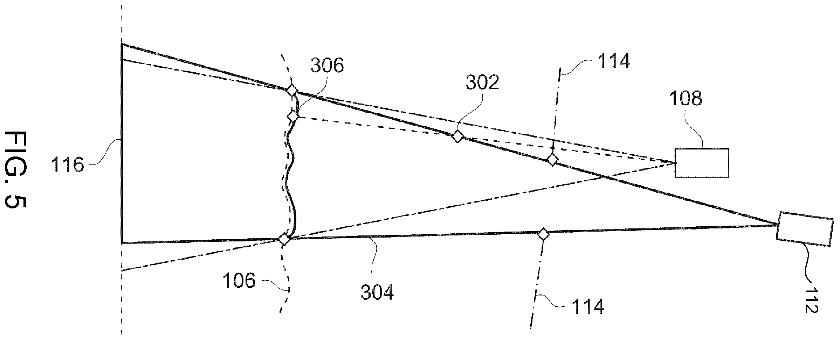

Fig. 5 depicts a schematic of a calculating 100.2 of 3D projection information 124.

Fig. 5 shows the camera system 108 and the x-ray tube 112, wherein an x-ray origin point 300.1 defines an origin of a coordinate system of the x-ray tube 112, and wherein the x-ray origin point 300.1 corresponds to the position of the x-ray tube. In an alternative example, a coordinate system of the collimator 114 may be used instead of the coordinate system of the x-ray tube 112, wherein points defined in the coordinate system of the collimator 114 may be shifted and/or rotated to align with the coordinate system of the x-ray tube by the addition of an offset vector, wherein the offset vector indicates a spatial displacement between the x-ray tube 112 and the collimator 114. Furthermore, a camera system origin point 300.2 defines an origin of a coordinate system of the camera system 108, wherein the camera system origin point corresponds to a position of the camera system 108. Fig. 5 further shows a collimator plane 114 corresponding to locations of the collimation blades of the collimator 114, wherein the collimator plane 114 limits the virtual lines 304 originating from the x-ray origin point 300.1 and extending towards the subject 106 positioned between the x-ray tube 112 and the detector 116. The virtual lines 304 correspond to possible paths of x-rays originating from the x-ray tube. Fig. 5 shows a 3D test point 302 on a virtual line 304, wherein a projection of the 3D test point 302 from the camera system origin point 300.2 results in a 3D camera projection point 306. While Fig. 5 shows the camera system 108 to be aligned perpendicular to the detector 116, the camera system 108 may alternatively be oriented at an angle relative to the detector 116. The x-ray tube 112 may be oriented at an angle relative to the camera system 108 as shown in Fig. 5.

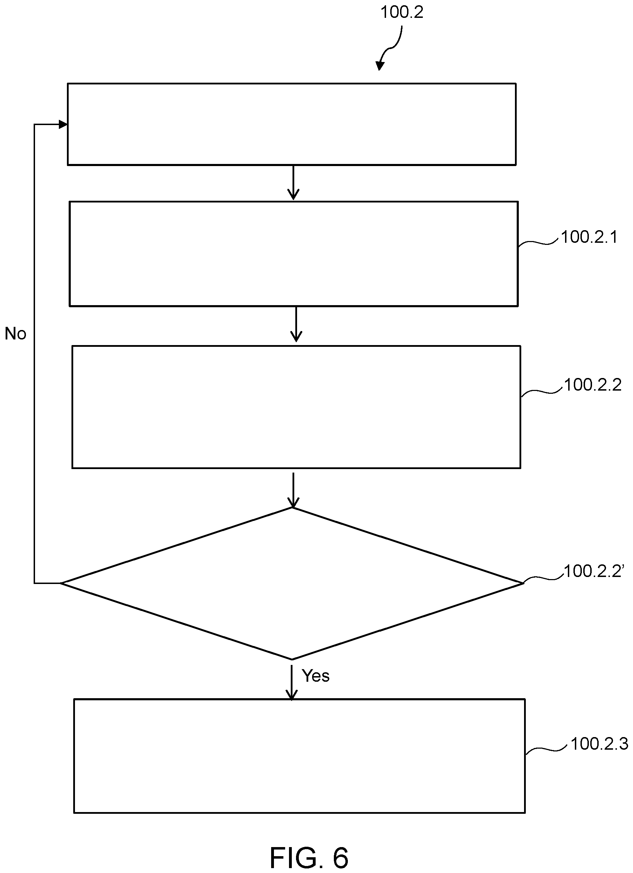

Fig. 6 shows a flowchart of a projection algorithm for implementing the calculating 100.2 of the 3D projection information 124.

In one example, the calculating 100.2 of the 3D projection information 124 further comprises defining an x-ray origin point 300.1 of an x-ray projection and a camera system origin point 300.2, the x-ray origin point 300.1 being a position of one of the x-ray components 110 at the position described by the position information 126 and the camera system origin point being a position of the camera system 108. According to this example, 3D test points 302 are located on virtual lines 304 originating from the x-ray origin point 300.1 and extend towards the subject support 104. According to this example, the calculating of the 3D projection information 124 further comprises for the 3D test points 302: performing 100.2.1 for the 3D test point 302 the transformation from the coordinate system of the x-ray tube 112 or collimator 114 to the coordinate system of the camera system 108, projecting 100.2.2 the transformed 3D test point 302' from the camera system origin point 300.2 towards the position of the subject 106 on the subject support 104 using the image data 120, resulting in a 3D camera projection point 306, and providing 100.2.3 the 3D camera projection point 306 as part of the 3D projection information 124 specifying the position of the subject 106 on the subject support 104 in case the 3D camera projection point 306 and the transformed 3D test point 302' are equal.

In the following, the term "z-axis" is used to refer to an axis pointing from the camera system 108 towards the subject support 104, and wherein a "z-coordinate" indicates a distance of a point towards the camera system 108 along the z-axis. The terms "x-axis" and "y-axis" are used to refer to orthogonal axes in a plane parallel to the subject support 104. In one example, the calculating 100.2 of the projection information may be performed by the computer system 702 of the x-ray system 102. According to said example, the calculating 100.2 of the projection information may begin by defining a 3D test point 302 on a virtual line 304 in three-dimensional space, wherein coordinates of the 3D test point 302 may be stored in a memory unit 705 of the computer system 702 of the x-ray system. The 3D test point 302 is then moved by a fixed step length along the virtual line 304, wherein the fixed step length may exemplarily comprise a three-dimensional vector of a fixed length, wherein the direction of the three-dimensional vector corresponds to a direction of the virtual line 304 pointing from the x-ray origin point 300.1 towards the subject 106.

In step 100.2.1, the coordinates representing the 3D test point 302 in the coordinate system of the x-ray tube 112 are transformed into coordinates representing the 3D test point 302 as a transformed 3D test point 302' in the coordinate system of the camera system 108. In step 100.2.2, the transformed 3D test point 302' is projected outward from the origin point 300.2 of the coordinate system of the camera system 108 towards the subject 106 on the subject support 104 by replacing a z-coordinate of the transformed 3D test point 302' by depth information 120.2 of the image data 120 provided by the camera system 108, wherein the depth information 120.2 is chosen from a pixel of the two-dimensional image of the visual information 120.1 corresponding to a direction of the virtual line 304 on which the transformed 3D test point 302' is located. The replacing of the z-coordinate results in an obtaining of a 3D camera projection point 306 from the transformed 3D test point 302'.

In a subsequent step 100.2.2', the 3D camera projection point 306 is compared to the transformed 3D test point 302', wherein the comparing exemplarily comprises determining a Euclidean distance between the 3D camera projection point 306 and the transformed 3D test point 302'. The 3D camera projection point 306 and the transformed 3D test point 302' may exemplarily be considered to be equal in case that the Euclidean distance is below a distance threshold, wherein the distance threshold may in one example be defined as a fixed percentage of the length of the fixed step length, for example 1%, 2%, or 5% of the length of the fixed step length. In another example, the distance threshold may be defined as a fixed length, for example 1 mm, 5 mm, or 1 cm. In case that the 3D camera projection point 306 and the transformed 3D test point 302' are determined to be equal, the 3D camera projection point 306 is provided in step 100.2.3 as part of the 3D depth information 124 specifying the position of the subject 106 on the subject support 104. In case that the 3D camera projection point 306 and the transformed 3D test point 302' are determined to not be equal, a new 3D test point 302 is defined by moving the 3D test point 302 along the virtual line 304 by the fixed step length, wherein the steps 100.2.1 and 100.2.2 and the comparison of the 3D camera projection point 306 and the transformed 3D test point 302' are repeated using the new 3D test point 302 as the 3D test point 302.

In one example, the operator 500 may indicate a two-dimensional position on the augmented image data 128 as input. The projection algorithm according to steps 100.2.1 to 100.2.3 is performed resulting in a 3D camera projection point 306 on the subject 106, wherein the 3D camera projection point 306 corresponds to the position on the augmented image data 128 indicated by the operator 500. The 3D camera projection point 306 is then projected towards the x-ray origin point 300.1, wherein the x-ray origin point may correspond to an actual position of the x-ray tube 112 or a virtual position of the x-ray tube 112. In case that the projection towards the x-ray origin point 300.1 intersects the collimation plane 114 in a collimator intersection point, the computer system 702 may calculate required translational and/or rotational movements of the x-ray tube 112 and/or the collimator 114 and/or a required opening of the collimation blades of the collimator 114, such that x-rays originating from the x-ray tube 112 are able to pass through the 3D camera projection point. The required opening of the collimation blades of the collimator 114 may for example be an asymmetrical opening of a single collimation blade or a symmetrical opening of two collimation blades. In one example, the opening of a particular collimation blade of the collimation is decided based on a distance of the particular collimation blade to the collimator intersection point.

The projection algorithm shown in Fig. 6 may be performed by the computer system 702 of the x-ray system to enable the calculation 100.2 of the 3D projection information 124 by the computer system 702. The computer system 702 may subsequently use the 3D projection information for determining the virtual positions 138 of the x-ray components corresponding to the virtual collimation fields 136. The 3D projection information may be used for determining the virtual positions 138 of the x-ray components 110, and in particular the virtual amplimat fields 200 associated with the virtual positions 138 of the detector 116.

In one example, the camera system 108 comprises a 3D camera rigidly mounted to the x-ray tube 112 or collimator 114, and the transformation from the coordinate system of the x-ray tube 112 or collimator 114 to the coordinate system of the camera system 108 for the 3D test point 302 is performed using a hand-eye calibration matrix 400. According to this example, the hand-eye calibration matrix 400 describes a rotation and/or translation between the position of the camera system 108 and the position of the x-ray components 110, and the hand-eye calibration matrix 400 is determined using the position information 126 and 3D calibration information. According to this example, the 3D calibration information comprises image data of the detector 116 in the camera image zone 122.

The hand-eye calibration matrix 400 may be obtained in a hand-eye calibration step before the performing of the method 100. The hand-eye calibration matrix 400 may be stored in the memory unit 705 of the computer system after the hand-eye calibration step. The hand-eye calibration step may exemplarily comprise an aligning of the coordinate system of the camera system 108 with the detector 116, wherein the position information 126 provides a position of the detector. The aligning may include a detection of a cover print pattern on the detector 116, for example a detecting of circular shapes indicating the amplimat fields 200 as shown in Fig. 1. The hand-eye calibration matrix may exemplarily comprise a 4 x 4 homogeneous matrix comprising a rotation matrix and a translation matrix.

In one example, the camera system 108 comprises a 3D camera rigidly mounted to the x-ray tube 112 or collimator 114, and the projecting of the transformed 3D test point 302' towards the position of the subject 106 on the subject support 104 is performed using a camera matrix 402, wherein the camera matrix 402 transforms the image data 120 to points in the coordinate system of the camera system 108 and is determined based on a pinhole camera model.

For example, the pinhole camera model comprises a pinhole plane at a lens of the camera system 108 and a projection plane at a sensor plate of the camera system 108, wherein a 2D pixel position comprising an x-coordinate and a y coordinate on a 2D pixel matrix of the camera system 108 corresponds to a particular point on the sensor plate of the camera system 108. The camera matrix 402 may then relate the x-coordinate and the y-coordinate of the 2D pixel position corresponding to the point on the sensor plate to an x-coordinate and a y-coordinate of a pixel in a two-dimensional image of the visual information 120.1 of the image data 120 using the z-coordinate provided by the depth information 120.2, wherein the z-coordinate is associated with the visual information 120.1. A use of the camera matrix 402 comprises both matrix multiplications with the camera matrix 402 as well as matrix multiplications with an inverse of the camera matrix 402.

In one example, the moving 100.6 of the x-ray components 110 to the sets of virtual positions 138 is performed using at least one combined transformation matrix 404. According to this example, the at least one combined transformation matrix 404 is determined using a matrix multiplication of the hand-eye calibration matrix 400 and a virtual transformation matrix 406, wherein the virtual transformation matrix 406 describes a rotation and/or translation between the positions of the x-ray components 110 and the virtual positions 138 of the x-ray components 110. The computer system 702 may be configured to send movement commands to the x-ray components based on the at least one combined transformation matrix 404, wherein the movement commands result in a movement of the x-ray components to a particular virtual position 138 associated with a particular virtual transformation matrix 406.

In one example, the display unit 130 comprises a touch screen interface mounted on the x-ray tube 112 as shown in Fig. 1, and the imaging instructions 132 may be received from the operator 500 using the touch screen interface. According to this example, the operator 500 may touch a point on the display unit 130, for example using a finger of the operator 500 or a stylus, followed by a second touch or a dragging motion, wherein the second touch or the dragging motion may define the target radiation field 134 as an overlay on the augmented image data 128. In a further example, the display unit may be a screen separate from the x-ray tube 112, wherein the screen is further configured to receive mouse commands. According to this example, the imaging instructions 132 may be received by the computer system 702 from the operator 500 as computer mouse click commands and/or dragging operations using a computer mouse.

The operator may exemplarily activate or deactivate virtual amplimat fields 200 for any of the virtual collimation fields 128 shown on the display unit 130, for example by clicking on highlighted areas of a virtual collimation field 136 corresponding to an area of a virtual amplimat field 200 using a computer mouse. The highlighted areas corresponding to the area of the amplimat fields 200 may exemplarily be displayed as overlays resulting from projecting rings in front of the detector 116, for example on a cover of the detector support system 116, onto the subject support 104 with respect to a center point of the x-ray tube 112, wherein a mouse click or touch screen input inside the rings activates a deactivated virtual amplimat field 200 or deactivates an activated virtual amplimat field 200.

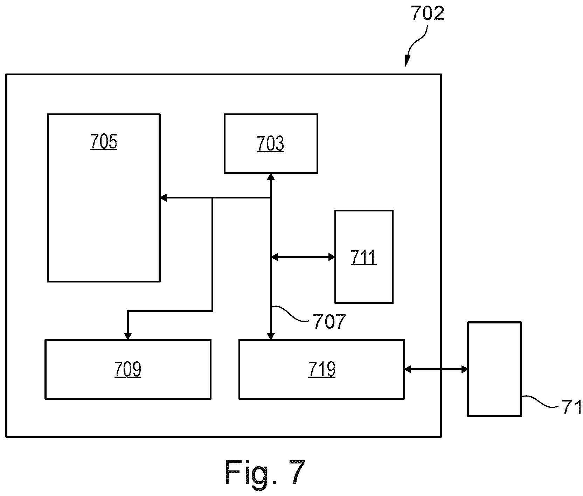

Fig. 7 shows a block diagram of an exemplary computer system for implementing the present method in accordance with an example of the present subject matter.

According to one example, the computer system may be communicatively coupled to the x-ray system 102, in particular to the display unit 132. According to another example, the computer system 702 may be integrated into the x-ray system, in particular, the computer system 702 may be integrated into the x-ray tube 112.

The components of the computer system 702 may include, but are not limited to, one or more processors or processing units 703, a storage system 711, a memory unit 705, and a bus 707 that couples various system components including memory unit 705 to processor 703. The storage system 711 may include for example a hard disk drive (HDD). The memory unit 705 may include computer system readable media in the form of volatile memory, such as random access memory (RAM) and/or cache memory.

The computer system 702 may also communicate with one or more external devices such as a keyboard, a pointing device, a display 713, etc.; one or more devices that enable a user to interact with computer system 702; and/or any devices (e.g., network card, modem, etc.) that enable the computer system 702 to communicate with one or more other computing devices. Such communication can occur via I/O interface(s) 719. Still yet, the computer system 702 can communicate with one or more networks such as a local area network (LAN), a general wide area network (WAN), and/or a public network (e.g., the Internet) via a network adapter 709. As depicted, the network adapter 709 communicates with the other components of the client system 702 via bus 707.

According to one example, the external devices 713 of the computer system 702 comprise the x-ray tube 112, the collimator 114, the detector 116 and/or the camera system 108 of the x-ray system 102. According to one example, the external devices 713 of the computer system further comprise a display 713 of the computer system 702, wherein the display 713 comprises the display unit 130 of the x-ray system 102.

The memory unit 705 is configured to store applications that are executable on the processor 703. For example, the memory unit 705 may comprise an operating system as well as one or more application programs. The application programs comprise instructions that when executed enable to perform the method described with reference to Fig. 2.

As will be appreciated by one skilled in the art, aspects of the present invention may be embodied as an apparatus, method, computer program or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon. A computer program comprises the computer executable code or "program instructions".

The term "computer system" refers to data processing hardware and encompasses all kinds of apparatus, devices, and machines for processing data, including by way of example, a programmable processor, a computer, or multiple processors or computers. The apparatus can also be or further include special purpose logic circuitry, e.g., a central processing unit (CPU), a FPGA (field programmable gate array), or an ASIC (application specific integrated circuit). In some implementations, the data processing apparatus and/or special purpose logic circuitry may be hardware-based and/or software-based. The apparatus can optionally include code that creates an execution environment for computer programs, e.g., code that constitutes processor firmware, a protocol stack, a database management system, an operating system, or a combination of one or more of them. The present disclosure contemplates the use of data processing apparatuses with or without conventional operating systems, for example LINUX, UNIX, WINDOWS, MAC OS, ANDROID, IOS or any other suitable conventional operating system.

Any combination of one or more computer readable medium(s) may be utilized. The computer readable medium may be a computer readable storage medium. A `computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device.

`Computer memory' or 'memory' is an example of a computer-readable storage medium. Computer memory is any memory which is directly accessible to a processor. `Computer storage' or 'storage' is a further example of a computer-readable storage medium. Computer storage is any nonvolatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

Generally, the program instructions can be executed on one processor or on several processors. In the case of multiple processors, they can be distributed over several different entities. Each processor could execute a portion of the instructions intended for that entity. Thus, when referring to a system or process involving multiple entities, the computer program or program instructions are understood to be adapted to be executed by a processor associated or related to the respective entity.

The described advantages of the inventive method equally apply to the inventive x-ray system and the inventive computer program product.

It is understood that one or more of the aforementioned examples may be combined as long as the combined examples are not mutually exclusive.

While the invention has been illustrated and described in detail in the drawings and foregoing description, such illustration and description are to be considered illustrative or exemplary and not restrictive; the invention is not limited to the disclosed examples.

### REFERENCE SIGNS LIST

- 100: method
- 102: x-ray system
- 104: subject support
- 106: subject
- 108: camera system
- 110: x-ray components
- 112: x-ray tube
- 114: collimator
- 116: detector
- 118: collimation field
- 120: image data
- 120.1: visual information
- 120.2: depth information
- 122: camera image zone
- 124: 3D depth information
- 126: position information
- 126.1: location information
- 126.2: orientation information
- 128: augmented image data
- 130: display unit
- 132: imaging instructions
- 134: target radiation field
- 136: virtual collimation field(s)
- 138: virtual positions
- 140: x-ray dataset
- 142, 142': (stitched) x-ray image
- 100.1-100.9: method steps
- 100.2.1-100.2.3: method steps
- 200: amplimat fields
- 300.1, 300.2: x-ray origin point, camera system origin point
- 302, 302': (transformed) 3D test points
- 304: virtual lines
- 306: 3D camera projection point
- 400: hand-eye calibration matrix
- 402: camera matrix
- 404: combined transformation matrix
- 406: virtual transformation matrix
- 500: operator
- 702: computer system
- 703: processors
- 705: memory unit
- 707: bus
- 709: network adapter
- 711: storage system
- 713: external devices
- 719: I/O-Interface

## Claims

1. A method (100) for controlling an x-ray system (102),
the x-ray system (102) comprising a subject support (104) configured for receiving a subject (106), a camera system (108) and x-ray components (110) comprising an x-ray tube (112), a collimator (114) mounted on the x-ray tube (112) and a detector (116), the x-ray system (102) defining a collimation field (118),
wherein the x-ray components (110) are configured to move in a planar translational motion along a plane defined by the subject support (104), wherein the x-ray tube (112) and the collimator (114) are further configured to move in a rotational motion around an axis parallel to the plane such that the collimation field (118) is also translated along the plane,
wherein the camera system (108) is configured to provide image data (120) of at least part of the subject support (104) in a camera image zone (122) larger than the collimation field (118),
the method (100) comprising:
receiving (100.1) the image data (120), wherein the image data comprises visual information (120.1) and depth information (120.2);
calculating (100.2) 3D projection information (124) specifying a position of the subject (106) on the subject support (104) relative to a position of the x-ray components (110) using the depth information (120.2), the position of the x-ray components (110) being described by position information (126);
providing (100.3) augmented image data (128) on a display unit (130), the augmented image data (128) comprising images rendered from the visual information (120.1) and the 3D projection information (124);
receiving (100.4) imaging instructions (132) from the display unit (130), the imaging instructions (132) defining a target radiation field (134);
determining (100.5) one or multiple virtual collimation fields (136) covering together the target radiation field (134), the target radiation field (134) being equal in size or larger than an individual one of the virtual collimation fields (136), wherein each virtual collimation field (136) is associated with a set of virtual positions (138) of the x-ray components (110) based on the 3D projection information (124) and the position information (126); and
moving (100.6) the x-ray components (110) in the planar translational motion and moving the x-ray tube (112) and the collimator (114) in the rotational motion to the sets of virtual positions (138).

2. The method (100) according to claim 1, the method further comprising:
for the sets of virtual positions (138), performing (100.7) an x-ray scan of the subject (106) using the virtual collimation field (136) actually defined by the set of virtual positions (138), the x-ray scan resulting in a respective x-ray dataset (140) per set of virtual positions (138),
constructing (100.8) at least one x-ray image (142) of the subject (106) based on the x-ray datasets (140) and the sets of virtual positions (138); and
providing (100.9) the at least one x-ray image (142).

3. The method (100) according to claims 1 or 2, wherein the position information (126) comprises location information (126.1) providing coordinates in 3D space and orientation information (126.2) providing angles of orientation of the x-ray components (110).

4. The method (100) according to claims 2 or 3, wherein the imaging instructions (132) further comprise instructions for activating or deactivating amplimat fields (200) of the detector (116) for the virtual collimation fields (136), wherein the x-ray scan is performed only using activated amplimat fields (200).

5. The method (100) according to any one of the preceding claims, wherein the moving (100.6) of the x-ray components (110) to the virtual positions (138) comprises moving the x-ray tube (112) in a planar translational motion to a central position of the target radiation field (134), maintaining the position of the x-ray tube (112) at the central position of the target radiation field (134), the moving (100.6) further comprising for the sets of virtual positions (138):
moving the detector (116) in a planar translational motion to the respective actual virtual position (138) of the detector (116);
rotating the x-ray tube (112) and the collimator (114) to align with the virtual collimation field (136) actually defined by the set of virtual positions (138).

6. The method (100) according to any one of the preceding claims, wherein the image data (120) comprises an RGB video data stream, in particular a live video feed, and the camera system (108) comprises a 3D camera or a set of 2D cameras.

7. The method (100) according to any one of the preceding claims, wherein the calculating (1002) of the 3D projection information (124) further comprises defining an x-ray origin point (300.1) of an x-ray projection and a camera system origin point (300.2), the x-ray origin point (300.1) being a position of one of the x-ray components (110) at the position described by the position information (126) and the camera system origin point being a position of the camera system (108), wherein 3D test points (302) are located on virtual lines (304) originating from the x-ray origin point (300.1) and extending towards the subject support (104), the calculating of the 3D projection information (124) further comprising for the 3D test points (302):
performing (100.2.1) for the 3D test point (302) the transformation from the coordinate system of the x-ray tube (112) or collimator (114) to the coordinate system of the camera system (108);
projecting (100.2.2) the transformed 3D test point (302') from the camera system origin point (300.2) towards the position of the subject (106) on the subject support (104) using the image data (120), resulting in a 3D camera projection point (306); and
providing (100.2.3) the 3D camera projection point (306) as part of the 3D projection information (124) specifying the position of the subject (106) on the subject support (104) in case the 3D camera projection point (306) and the transformed 3D test point (302') are equal.

8. The method (100) according to claim 7, the 3D camera (108) being rigidly mounted to the x-ray tube (112) or collimator (114), wherein the transformation from the coordinate system of the x-ray tube (112) or collimator (114) to the coordinate system of the camera system (108) for the 3D test point (302) is performed using a hand-eye calibration matrix (400), wherein the hand-eye calibration matrix (400) describes a rotation and/or translation between the position of the camera system (108) and the position of the x-ray components (110), and wherein the hand-eye calibration matrix (400) is determined using the position information (126) and 3D calibration information, wherein the 3D calibration information comprises image data of the detector (116) in the camera image zone (122).

9. The method (100) according to claim 7 or 8, the 3D camera (108) being rigidly mounted to the x-ray tube (112) or collimator (114), wherein the projecting of the transformed 3D test point (302') towards the position of the subject (106) on the subject support (104) is performed using a camera matrix (402), wherein the camera matrix (402) transforms the image data (120) to points in the coordinate system of the camera system (108) and is determined based on a pinhole camera model.

10. The method (100) according to claims 5 through 9, wherein the moving (100.6) of the x-ray components (110) to the sets of virtual positions (138) is performed using at least one combined transformation matrix (404), wherein the at least one combined transformation matrix (404) is determined using a matrix multiplication of the hand-eye calibration matrix (400) and a virtual transformation matrix (406), wherein the virtual transformation matrix (406) describes a rotation and/or translation between the positions of the x-ray components (110) and the virtual positions (138) of the x-ray components (110).

11. The method (100) according to any one of the preceding claims, wherein the imaging instructions (132) are received from an operator (500) entering commands on a computer mouse, and/or wherein the display unit (130) comprises a touch screen interface, and wherein the imaging instructions (132) are received from the operator (500) using the touch screen interface.

12. The method (100) according to any one of the claims 2 through 11, wherein the constructing (100.8) of the x-ray image (142) comprises a stitching of multiple x-ray images (142) resulting from the performing of multiple x-ray scans on multiple collimation fields (118) covering the target radiation field (134) into a stitched x-ray image (142'), and providing the x-ray image (142) as the stitched x-ray image (142').

13. The method (100) according to any one of the preceding claims, wherein the virtual collimation fields (136) cover the target radiation field (134) in a contiguous and/or overlapping manner, and/or wherein a size of the target radiation field (134) exceeds a size of the detector (116).

14. An x-ray system (102),
the x-ray system (102) comprising a subject support (104) configured for receiving a subject (106), a camera system (108) and x-ray components (110) comprising an x-ray tube (112), a collimator (114) mounted on the x-ray tube (112) and a detector (116), the x-ray system (102) defining a collimation field (118),
wherein the x-ray components (110) are configured to move in a planar translational motion along a plane defined by the subject support (104), wherein the x-ray tube (112) and the collimator (114) are further configured to move in a rotational motion around an axis parallel to the plane such that the collimation field (118) is also translated along the plane,
wherein the camera system (108) is configured to provide image data (120) of at least part of the subject support (104) in a camera image zone (122) larger than the collimation field (118),
the x-ray system (102) further comprising a computer system (702), the computer system (702) comprising a memory unit (705) storing machine executable instructions, wherein execution of the machine executable instructions causes the computer system (702) to:
receive (100.1) the image data (120), the image data comprising visual information (120.1) and depth information (120.2);
calculate (100.2) 3D projection information (124) specifying a position of the subject (106) on the subject support (104) relative to a position of the x-ray components (110) using the depth information (120.2), the position of the x-ray components (110) being described by position information (126);
provide (100.3) augmented image data (128) on a display unit (130), the augmented image data (128) comprising images rendered from the visual information (120.1) and the 3D projection information (124);
receive (100.4) imaging instructions (132) from the display unit (130), the imaging instructions (132) defining a target radiation field (134);
determine (100.5) one or multiple virtual collimation fields (136) covering together the target radiation field (134), the target radiation field (134) being equal in size or larger than an individual one of the virtual collimation fields (136), wherein each virtual collimation field (136) is associated with a set of virtual positions (138) of the x-ray components (110) based on the 3D projection information (124) and the position information (126); and
move (100.6) the x-ray components (110) in the planar translational motion and move the x-ray tube (112) and the collimator (114) in the rotational motion to the sets of virtual positions (138).

15. A computer program product comprising machine executable instructions configured for causing an x-ray system (102) to perform the method (100) of claims 1 through 13.
